# EUROPEAN PATENT APPLICATION

(11) **EP 1 069 134 A1**
(43) Date of publication of application: **17.01.2001**
(21) Application number: 99305620.9
(22) Date of filing: 15.07.1999
(51) Int. Cl.: C07K 14/24, C12P 21/02

(54) **Photorhabdus luminescens strains**

(71) Applicant: WISCONSIN ALUMNI RESEARCH FOUNDATION, Madison, WI 53705 (US)
(72) Inventor: The designation of the inventor has not yet been filed
(74) Representative: Raynor, John

(57) **Abstract**

Use of novel microorganisms from the genus *Photorhabdus* to produce protein toxins which are toxic to insects upon exposure and can be applied to, or genetically engineered into, insect larvae food and plants for insect control.

## Description

WO 98/08932 and WO 97/17432 describe the utility of *Photorhabdus luminescens* bacteria as producers of protein toxins that are active against insects upon ingestion. A number of specific *Photorhabdus luminescens* strains were named and described in WO 98/08932 and WO 97/17432, but International Depository accession numbers were not included in the published applications for many of the strains.

The present invention provides use of a culture of one of the following isolated *Photorhabdus luminescens* strains to produce a protein toxin having functional activity against insects:

| strain name | accession number |
|---|---|
| WX1 | NRRL B-21710 |
| WX2 | NRRL B-21711 |
| WX3 | NRRL B-21712 |
| WX4 | NRRL B-21713 |
| WX5 | NRRL B-21714 |
| WX6 | NRRL B-21715 |
| WX7 | NRRL B-21716 |
| WX8 | NRRL B-21717 |
| WX9 | NRRL B-21718 |
| WX10 | NRRL B-21719 |
| WX11 | NRRL B-21720 |
| WX12 | NRRL B-21721 |
| WX14 | NRRL B-21722 |
| WX15 | NRRL B-21723 |
| H9 | NRRL B-21727 |
| Hb | NRRL B-21726 |
| Hm | NRRL B-21725 |
| HP88 | NRRL B-21724 |
| NC-1 | NRRL B-21728 |
| W30 | NRRL B-21729 |
| WIR | NRRL B-21730 |
| B2 | NRRL B-21731 |
| P. zealandrica | NRRL B-21683 |
| P. hepialus | NRRL B-21684 |
| HB-Arg | NRRL B-21685 |
| HB Oswego | NRRL B-21686 |
| Hb Lewiston | NRRL B-21687 |
| K-122 | NRRL B-21688 |
| HMGD | NRRL B-21689 |
| Indicus | NRRL 5-21690 |
| GD | NRRL B-21691 |
| PWH-5 | NRRL B-21692 |
| Megidis | NRRL B-21693 |
| HF-85 | NRRL B-21694 |
| A. Cows | NRRL B-21695 |
| MP1 | NRRL B-21696 |
| MP2 | NRRL B-21697 |
| MP3 | NRRL B-21698 |
| MP4 | NRRL B-21699 |
| MP5 | NRRL B-21700 |
| GL98 | NRRL B-21701 |
| Gl101 | NRRL B-21702 |
| GL138 | NRRL B-21703 |
| GL155 | NRRL B-21704 |
| GL217 | NRRL B-21705 |
| GL257 | NRRL B-21706 |

All of the above listed strains were deposited on April 29, 1997 at United States Department of Agriculture, 1815 North University Drive, Peoria, IL 61604, and were assigned the accession numbers given in the foregoing table.

The invention also provides *Photorhabdus* protein toxins having functional activity against insects produced by culturing a microorganism selected from the group consisting of WX-1, WX-2, WX-3, WX-4, WX-5, WX6, WX-7, WX-8, WX-9, WX-10, WX-11, WX-12, WX-14, WX-15, H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, GL257.

The invention also provides insect control compositions comprising an effective amount of a *Photorhabdus* protein toxin produced by culturing a microorganism selected from the group consisting of WX-1, WX-2, WX-3, WX-4, WX-5, WX6, WX-7, WX-8, WX-9, WX-10, WX-11, WX-12, WX-14, WX-15, H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, and GL257 in combination with an agronomically acceptable carrier.

The invention also provides a purified culture of a microorganism selected from the group comprising H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, or GL257.

The invention also provides a method of controlling insects which comprises causing a prospective food source of a target insect to contain an effective amount of a *Photorhabdus* protein toxin that has functional activity and is produced by culturing a microorganism selected from the group consisting of WX-1, WX-2, WX-3, WX-4, WX-5, WX6, WX-7, WX-8, WX-9, WX-10, WX-11, WX-12, WX-14, WX-15, H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, and GL257.

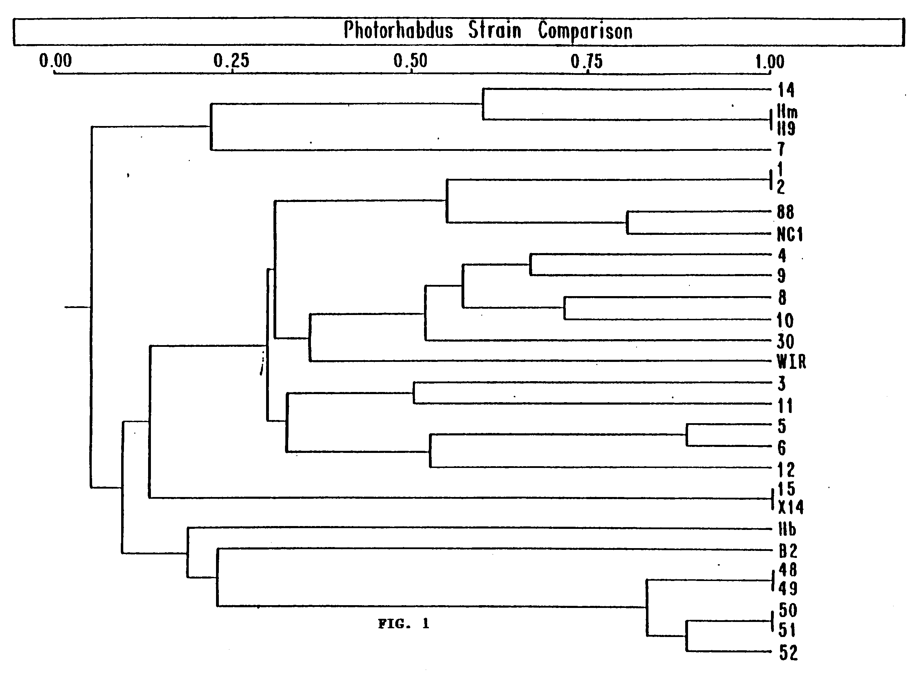

Fig. 1 is a phenogram of *Photorhabdus* strains. Relationship of *Photorhabdus* Strains was defined by rep-PCR.
The upper axis of Fig. 1 measures the percentage similarity of strains based on scoring of rep-PCR products (i.e., 0.0 [no similarity] to 1.0 [100% similarity]). At the right axis, the numbers and letters indicate the various strains tested; 14=W-14, Hm=Hm, H9=H9, 7=WX-7, 1=WX-1, 2=WX-2, 88=HP88, NC-1=NC-1, 4=WX-4, 9=WX-9, 8=WX-8, 10=WX-10, WIR=WIR, 3=WX-3, 11=WX-11, 5=WX-5, 6=WX-6, 12=WX-12, x14=WX-14, 15=WX-15, Hb=Hb, B2=B2, 48 through 52=ATCC 43948 through ATCC 43952. Vertical lines separating horizontal lines indicate the degree of relatedness (as read from the extrapolated intersection of the vertical line with the upper axis) between strains or groups of strains at the base of the horizontal lines (e.g., strain W-14 is approximately 60% similar to strains H9 and Hm).

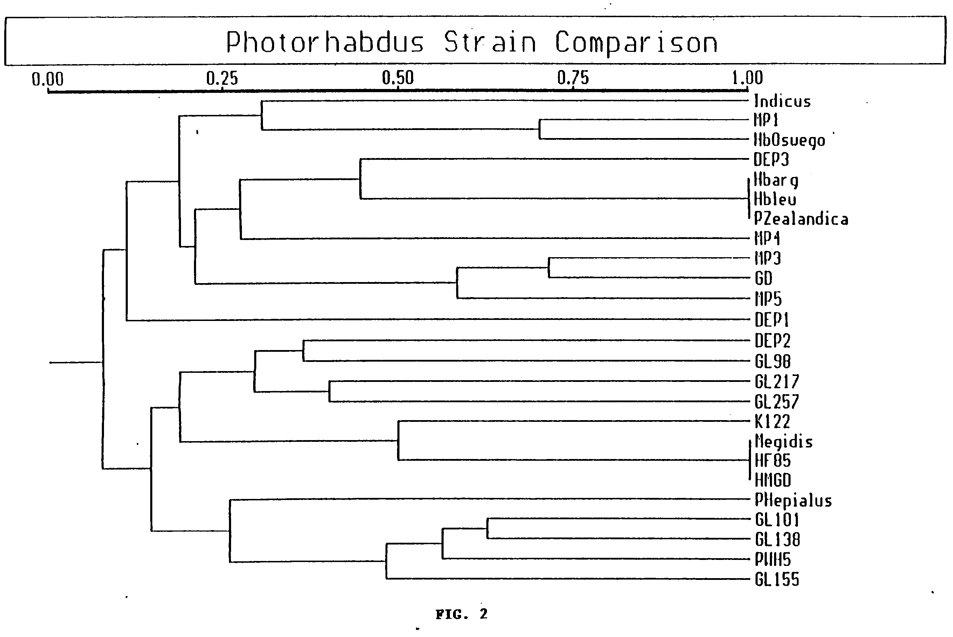

Fig. 2 is a phenogram of *Photorhabdus* strains as defined by rep-PCR. The upper axis of Fig. 2 measures the percentage similarity of strains based on scoring of rep-PCR products (i.e., 0.0 [no similarity] to 1.0 [100% similarity]). At the right axis, the numbers and letters indicate the various strains tested. Vertical lines separating horizontal lines indicate the degree of relatedness (as read from the extrapolated intersection of the vertical line with the upper axis) between strains or groups of strains at the base of the horizontal lines (e.g., strain Indicus is approximately 30% similar to strains MP1 and HB Oswego). Note that the *Photorhabdus* strains on the phenogram are as follows: 14 = W-14; Hm = Hm; H9 = H9; 7 = WX-7; 1 = WX-1; 2 = WX-2; 88 = HP88; NC1 = NC-1; 4 = WX-4; 9 = WX-9; 8 = WX-8; 10 = WX-10; 30 = W30; WIR = WIR; 3 - WX-3; 11 = WX-11; 5 = WX-5; 6 = WX-6; 12 = WX-12; 15 = WX-15; X14 = WX-14; Hb = Hb; B2 = B2; 48 = ATCC 43948; 49 = ATCC 43949; 50 = ATCC 43950; 51 = ATCC 43951; 52 = ATCC 43952.

For purposes of this application, designation of a toxin as having "functional activity" means that the protein functions as an insect control agent in that the protein is active upon ingestion. When an insect comes into contact with an effective amount of toxin delivered via transgenic plant expression, formulated protein compositions(s), sprayable protein composition(s), a bait matrix or other delivery system, the results are typically death of the insect, or the insects do not feed upon the source which makes the toxins available to the insects.

The protein toxins discussed herein are typically referred to as "insecticides". By insecticides it is meant herein that the protein toxins have a "functional activity" as further defined herein and are used as insect control agents.

The strains characterized herein have been shown to have toxicity upon ingestion by a variety of insect orders. Such insect orders include but are not limited to *Coleoptera, Homoptera, Lepidoptera, Diptera, Acarina, Hymenoptera* and *Dictyoptera.*

As with other bacterial toxins, the rate of mutation of the bacteria in a population causes many related toxins slightly different in sequence to exist. Toxins of interest here are those which produce protein complexes toxic to a variety of insects upon exposure, as described herein. Preferably, the toxins are active against *Lepidoptera, Coleoptera, Homopotera, Diptera, Hymenoptera, Dictyoptera* and *Acarina*. The inventions herein are intended to capture the protein toxins homologous to protein toxins produced by the strains herein and any derivative strains thereof. These homologous proteins may differ in sequence, but do not differ in function from those toxins described herein. Homologous toxins are meant to include protein complexes of between 300 kDa to 2,000 kDa and are comprised of at least two (2) subunits, where a subunit is a peptide which may or may not be the same as the other subunit. Various protein subunits have been identified and are taught in the Examples herein. Typically, the protein subunits are between about 18 kDa to about 230 kDa; between about 160 kDa to about 230 kDa; 100 kDa to 160 kDa; about 80 kDa to about 100 kDa; and about 50 kDa to about 80 kDa.

Using the procedure described in Example 12 of WO98/08932, strains were assesed in terms of recognized characteristic microbiological traits. The results are given in Table 1:

**Table 1**

| Taxonomic Traits of *Photorhabdus* Strains Traits Assessed* | | | | | | | | | | | | | | | | | |
|---|---|---|---|---|---|---|---|---|---|---|---|---|---|---|---|---|---|
| Strain | A | B | C | D | E | F | G | H | I | J | K | L | M | N | O | P | Q |
| WX-1 | - | ± | ± | rd S | ± | - | ± | ± | ± | O | ± | ± | ± | ± | ± | ± | - |
| WX-2 | - | ± | ± | rd S | ± | - | ± | ± | ± | O | ± | ± | ± | ± | ± | ± | - |
| WX-3 | - | ± | ± | rd S | ± | - | ± | ± | ± | YT | ± | ± | ± | ± | ± | ± | - |
| WX-4 | - | ± | ± | rd S | ± | - | ± | ± | ± | YT | ± | ± | ± | ± | ± | ± | - |
| WX-5 | - | ± | ± | rd S | ± | - | ± | ± | ± | LO | ± | ± | ± | ± | ± | ± | - |
| WX-6 | - | ± | ± | rd S | ± | - | ± | ± | ± | LY | ± | ± | ± | ± | ± | ± | - |
| WX-7 | - | ± | ± | rd S | ± | - | ± | ± | ± | R | ± | ± | ± | ± | ± | ± | - |
| WX-8 | - | ± | ± | rd S | ± | - | ± | ± | ± | O | ± | ± | ± | ± | ± | ± | - |
| WX-9 | - | ± | ± | rd S | ± | - | ± | ± | ± | YT | ± | ± | ± | ± | ± | ± | - |
| WX-10 | - | ± | ± | rd S | ± | - | ± | ± | ± | Ro | ± | ± | ± | ± | ± | ± | - |
| WX-11 | - | ± | ± | rd S | ± | - | ± | ± | ± | Ro | ± | ± | ± | ± | ± | ± | - |
| WX-12 | - | ± | ± | rd S | ± | - | ± | ± | ± | O | ± | ± | ± | ± | ± | ± | - |
| WX-14 | - | ± | ± | rd S | ± | - | ± | ± | ± | LR | ± | ± | ± | ± | ± | ± | - |
| WX-15 | - | ± | ± | rd S | ± | - | ± | ± | ± | LR | ± | ± | ± | ± | ± | ± | - |
| H9 | - | ± | ± | rd S | ± | - | ± | ± | ± | LY | ± | ± | ± | ± | ± | ± | - |
| Hb | - | ± | ± | rd S | ± | - | ± | ± | ± | YT | ± | ± | ± | ± | ± | ± | - |
| Hm | - | ± | ± | rd S | ± | - | ± | ± | ± | TY | ± | ± | ± | ± | ± | ± | - |
| HP88 | - | ± | ± | rd S | ± | - | ± | ± | ± | LY | ± | ± | ± | ± | ± | ± | - |
| NC-1 | - | ± | ± | rd S | ± | - | ± | ± | ± | O | ± | ± | ± | ± | ± | ± | - |
| W30 | - | ± | ± | rd S | ± | - | ± | ± | ± | YT | ± | ± | ± | ± | ± | ± | - |
| WIR | - | ± | ± | rd S | ± | - | ± | ± | ± | RO | ± | ± | ± | ± | ± | ± | - |
| B2 | - | ± | ± | rd S | ± | - | ± | ± | ± | R | ± | ± | ± | ± | ± | ± | - |

| | | | | | | | | | | | | | | | | | |
|---|---|---|---|---|---|---|---|---|---|---|---|---|---|---|---|---|---|
| * - A = Gram's stain, B=Crystaline inclusion bodies, C=Bioluminescence, D=Cell form, E=Motility, F=Nitrate reduction, G=Presence of catalase, H=Gelatin hydrolysis, I=Dye uptake, J=Pigmentation, K=Growth on EMB agar, L=Growth on MacConkey agar, M=Growth on Tergitol-7 agar, N=Facultative anaerobe, O=Growth at 20°C, P=Growth at 28°C, Q=Growth at 37°C, † - +/- = positive or negative for trait, rd=rod, S=sized within Genus descriptors, RO=red-orange, LR = light red, R= red, O= orange, Y= yellow, T= tan, LY= light yellow, YT= yellow tan, and LO= light orange. | | | | | | | | | | | | | | | | | |

As described on page 85 of WO98/08932, the evolutionary diversity of the *Photorhabdus* strains was measured by analysis of PCR (Polymerase Chain Reaction) mediated genomic fingerprinting using genomic DNA from each strain. The data from *Photorhabdus* strains were then analyzed with a series of programs within NTSYS-pc; SIMQUAL (Similarity for Qualitative data) to generate a matrix of similarity coefficients (using the Jaccard coefficient) and SAHN (Sequential, Agglomerative, Heirarchical and Nested) clustering [using the UPGMA (Unweighted Pair-Group Method with Arithmetic Averages) method] which groups related strains and can be expressed as a phenogram (Fig. 1). The COPH (cophenetic values) and MXCOMP (matrix comparison) programs were used to generate a cophenetic value matrix and compare the correlation between this and the original matrix upon which the clustering was based. A resulting normalized Mantel statistic (r) was generated which is a measure of the goodness of fit for a cluster analysis (r=0.8-0.9 represents a very good fit). In our case r = 0.919. Therefore, our collection is comprised of a diverse group of easily distinguishable strains representative of the *Photorhabdus* genus. Using the procedure described in Example 22 of WO98/08932, strains were assessed in terms of recognized characteristic microbiological traits. The results are given in Table 2:

**Table 2**

| Taxonomic Traits of *Photorhabdus* Strains | | | | | | | | | | | | | | | | | |
|---|---|---|---|---|---|---|---|---|---|---|---|---|---|---|---|---|---|
| Strain | A* | B | C | D | E | F | G | H | I | J^{§} | K | L | M | N | O | P | Q |
| P.zealandica | -† | + | + | rd S | + | - | + | + | + | PO | + | + | + | + | + | + | - |
| P. hepialus | - | + | + | rd S | + | - | + | + | + | Y | + | + | + | + | + | + | - |
| HB-Arg | - | + | + | rd S | + | - | + | + | + | W | + | + | + | + | + | + | - |
| HB Oswego | - | + | + | rd S | + | - | + | + | + | W | + | + | + | + | + | + | - |
| HB Lewiston | - | + | + | rd S | + | - | + | + | + | T | + | + | + | + | + | + | - |
| K-122 | - | + | + | rd S | + | - | + | + | + | Y | + | + | + | + | + | + | - |
| HMGD | - | + | + | rd S | + | - | + | + | + | Rd | + | + | + | + | + | + | - |
| Indicus | - | + | + | rd S | + | - | + | + | + | W | + | + | + | + | + | + | - |
| GD | - | + | + | rd S | + | - | + | + | + | YT | + | + | + | + | + | + | - |
| PWH-5 | - | + | + | rd S | + | - | + | + | + | Y | + | + | + | + | + | + | - |
| Megidis | - | + | + | rd S | + | - | + | + | + | R | + | + | + | + | + | + | - |
| HF-85 | - | + | + | rd S | + | - | + | + | + | R | + | + | + | + | + | + | - |
| A. Cows | - | + | + | rd S | + | - | + | + | + | PR | + | + | + | + | + | + | - |
| MP1 | - | + | + | rd S | + | - | + | + | + | T | + | + | + | + | + | + | - |
| MP2 | - | + | + | rd S | + | - | + | + | + | T | + | + | + | + | + | + | - |
| MP3 | - | + | + | rd S | + | - | + | + | + | R | + | + | + | + | + | + | - |
| MP4 | - | + | + | rd S | + | - | + | + | + | Y | + | + | + | + | + | + | - |
| MP5 | - | + | + | rd S | + | - | + | + | + | PR | + | + | + | + | + | + | - |
| GL98 | - | + | + | rd S | + | - | + | + | + | W | + | + | + | + | + | + | - |
| GL101 | - | + | + | rd S | + | - | + | + | + | W | + | + | + | + | + | + | - |
| GL138 | - | + | + | rd S | + | - | + | + | + | W | + | + | + | + | + | + | - |
| GL155 | - | + | + | rd S | + | - | + | + | + | W | + | + | + | + | + | + | - |
| GL217 | - | + | + | rd S | + | - | + | + | + | Y | + | + | + | + | + | + | - |
| GL257 | - | + | + | rd S | + | - | + | + | + | O | + | + | + | + | + | + | - |

| | | | | | | | | | | | | | | | | | |
|---|---|---|---|---|---|---|---|---|---|---|---|---|---|---|---|---|---|
| *: A=Gram's stain, B=Crystaline inclusion bodies, C=Bioluminescence, D=Cell form, E=Motility, F=Nitrate reduction, G=Presence of catalase, H=Gelatin hydrolysis, I=Dye uptake, J=Pigmentation on Nutrient Agar (some color shifts after Day 5), K=Growth on EMB agar, L=Growth on MacConkey agar, M=Growth on Tergitol-7 agar, N =Facultative anaerobe, O=Growth at 20°C, P=Growth at 28°C, Q=Growth at 37°C. | | | | | | | | | | | | | | | | | |
| †: +=positive for trait, - =negative for trait; rd=rod, S=sized within Genus descriptors. | | | | | | | | | | | | | | | | | |
| _{§}: W = white, CR = cream, Y =yellow, YT=yellow tan, T=tan PO=pale orange, O=orange, PR=pale red, R=red. | | | | | | | | | | | | | | | | | |

As described on page 129 of WO98/08932, the evolutionary diversity of this group of *Photorhabdus* strains was measured by analysis of PCR (Polymerase Chain Reaction) mediated genomic fingerprinting using genomic DNA from each strain. The data from *Photorhabdus* strains were then analyzed with a series of programs within NTSYS-pc; SIMQUAL (Similarity for Qualitative data) to generate a matrix of similarity coefficients (using the Jaccard coefficient) and SAHN (Sequential, Agglomerative, Heirarchical and Nested) clustering [using the UPGMA (Unweighted Pair-Group Method with Arithmetic Averages) method] which groups related strains and can be expressed as a phenogram (Fig. 2). The COPH (cophenetic values) and MXCOMP (matrix comparison) programs were used to generate a cophenetic value matrix and compare the correlation between this and the original matrix upon which the clustering was based. A resulting normalized Mantel statistic (r) was generated which is a measure of the goodness of fit for a cluster analysis (r=0.8-0.9 represents a very good fit). In our case r=0.924. Therefore, this group is also comprised of a diverse group of easily distinguishable strains representative of the *Photorhabdus* genus.

Using the test procedures described on pages 87-93 of WO 98/08932, the insecticidal activity of toxins produced by various Photorhabdus strains was evaluated. The results are given in the following Table 3:

**Table 3**

| Observed Insecticidal Spectrum of Broths from Different *Photorhabdus* Strains | |
|---|---|
| *Photorhabdus* Strain | Sensitive* Insect Species |
| WX-1 | 3**, 4, 5, 6, 7, 8 |
| WX-2 | 2, 4 |
| WX-3 | 1, 4 |
| WX-4 | 1, 4 |
| WX-5 | 4 |
| WX-6 | 4 |
| WX-7 | 3, 4, 5, 6, 7, 8 |
| WX-8 | 1, 2, 4 |
| WX-9 | 1, 2, 4 |
| WX-10 | 4 |
| WX-11 | 1, 2, 4 |
| WX-12 | 2, 4, 5, 6, 7, 8 |
| WX-14 | 1, 2, 4 |
| WX-15 | 1, 2, 4 |
| W30 | 3, 4, 5, 8 |
| NC-1 | 1, 2, 3, 4, 5, 6, 7, 8, 9 |
| WIR | 2, 3, 5, 6, 7, 8 |
| HP88 | 1, 3, 4, 5, 7, 8 |
| Hb | 3, 4, 5, 7, 8 |
| Hm | 1, 2, 3, 4, 5, 7, 8 |
| H9 | 1, 2, 3, 4, 5, 6, 7, 8 |

| | |
|---|---|
| * = ≥ 25% mortality and/or growth inhibition vs. control | |
| ** = 1; Tobacco budworm, 2; European corn borer, 3; Tobacco hornworm, 4; Southern corn rootworm, 5; Boll weevil, 6; Mosquito, 7; Fruit Fly, 8; Aster Leafhopper, 9; Corn planthopper, 10; Two-spotted spider mite. | |

Using the test procedures described on pages 132-136 of WO 98/08932, the insecticidal activity of toxins produced by various Photorhabdus strains was evaluated. The results are given in the following Table 4:

**Table 4**

| Observed Insecticidal Spectrum of Broths from Different *Photorhabdus* Strains | |
|---|---|
| *Photorhabdus* Strain | Sensitive* Insect Species |
| P. zealandica | 1**, 2, 4 |
| P. hepialus | 1, 2, 4 |
| HB-Arg | 1, 2, 4 |
| HB Oswego | 1, 2, 4 |
| HB Lewiston | 1, 2, 4 |
| K-122 | 1, 4 |
| HMGD | 1, 4 |
| Indicus | 1, 2, 4 |
| GD | 2, 4 |
| PWH-5 | 1, 2, 4 |
| Megidis | 1, 2, 4 |
| HF-85 | 1, 2, 4 |
| A. Cows | 1, 4 |
| MP1 | 1, 2, 4 |
| MP2 | 1, 2, 4 |
| MP3 | 4 |
| MP4 | 1, 4 |
| MP5 | 4 |
| GL98 | 1, 4 |
| GL101 | 1, 4, 5 |
| GL138 | 1, 2, 4 |
| GL155 | 1, 4 |
| GL217 | 1, 2, 4 |
| GL257 | 1, 4 |

| | |
|---|---|
| * = = 25% mortality and/or growth inhibition vs. control | |
| ** = 1; Tobacco budworm, 2; European corn borer, 3; Tobacco hornworm, 4; Southern corn rootworm, 5; German cockroach. | |

## Claims

1. Use of a culture of a microorganism strain selected from the group consisting of
| strain name | accession number |
|---|---|
| WX1 | NRRL B-21710 |
| WX2 | NRRL B-21711 |
| WX3 | NRRL B-21712 |
| WX4 | NRRL B-21713 |
| WX5 | NRRL B-21714 |
| WX6 | NRRL B-21715 |
| WX7 | NRRL B-21716 |
| WX8 | NRRL B-21717 |
| WX9 | NRRL B-21718 |
| WX10 | NRRL B-21719 |
| WX11 | NRRL B-21720 |
| WX12 | NRRL B-21721 |
| WX14 | NRRL B-21722 |
| WX15 | NRRL B-21723 |
| H9 | NRRL B-21727 |
| Hb | NRRL B-21726 |
| Hm | NRRL B-21725 |
| HP88 | NRRL B-21724 |
| NC-1 | NRRL B-21728 |
| W30 | NRRL B-21729 |
| WIR | NRRL B-21730 |
| B2 | NRRL B-21731 |
| P. zealandrica | NRRL B-21683 |
| P. hepialus | NRRL B-21684 |
| HB-Arg | NRRL B-21685 |
| HB Oswego | NRRL B-21686 |
| Hb Lewiston | NRRL B-21687 |
| K-122 | NRRL B-21688 |
| HMGD | NRRL B-21689 |
| Indicus | NRRL B-21690 |
| GD | NRRL B-21691 |
| PWH-5 | NRRL B-21692 |
| Megidis | NRRL B-21693 |
| HF-85 | NRRL B-21694 |
| A. Cows | NRRL B-21695 |
| MP1 | NRRL B-21696 |
| MP2 | NRRL B-21697 |
| MP3 | NRRL B-21698 |
| MP4 | NRRL B-21699 |
| MP5 | NRRL B-21700 |
| GL98 | NRRL B-21701 |
| Gl101 | NRRL B-21702 |
| GL138 | NRRL B-21703 |
| GL155 | NRRL B-21704 |
| GL217 | NRRL B-21705 |
| GL257 | NRRL B-21706 |
to produce a protein toxin having functional activity against insects.

2. Use of an isolated microorganism strain to produce a protein toxin having functional activity against insects as claimed in claim 1 wherein the strain is selected from the group consisting of WX-1, WX-2, WX-3, WX-4, WX-5, WX6, WX-7, WX-8, WX-9, WX-10, WX-11, WX-12, WX-14, and WX-15.

3. Use of an isolated microorganism strain to produce a protein toxin having functional activity against insects as claimed in claim 1 wherein the strain is selected from the group consisting of H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, and GL257.

4. A *Photorhabdus* protein toxin having functional activity against insects produced by culturing a microorganism selected from the group consisting of WX-1, WX-2, WX-3, WX-4, WX-5, WX6, WX-7, WX-8, WX-9, WX-10, WX-11, WX-12, WX-14, WX-15, H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, and GL257.

5. An insect control composition comprising an effective amount of a *Photorhabdus* protein toxin of claim 3 in combination with an agronomically acceptable carrier.

6. A purified culture of a microorganism selected from the group consisting of H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, and GL257.

7. A method of controlling insects which comprises causing a prospective food source of a target insect to contain an effective amount of a *Photorhabdus* protein toxin that has functional activity and is produced by culturing microorganism selected from the group consisting of WX-1, WX-2, WX-3, WX-4, WX-5, WX6, WX-7, WX-8, WX-9, WX-10, WX-11, WX-12, WX-14, WX-15, H9, Hb, Hm, HP88, NC-1, W30, WIR, B2, P. zealandrica, P. hepialus, HB-Arg, HB Oswego, HB Lewiston, K-122, HMGD, Indicus, GD, PWH-5, Megidis, HF-85, A. Cows, MP1, MP2, MP3, MP4, MP5, GL98, GL155, GL101, GL138, GL217, and GL257.
